# EUROPEAN PATENT APPLICATION

(11) **EP 2 226 391 A1**
(43) Date of publication of application: **08.09.2010**
(21) Application number: 09003292.1
(22) Date of filing: 06.03.2009
(51) Int. Cl.: C12Q 1/68

(54) **Paratuberculosis susceptibility test**

(71) Applicant: Ikonomopoulos, Joannis, 152 34 Halandri (GR)
(72) Inventor: Gazouli, Maria, 11633 Pagrati Athens (GR); Liandris, Emmanouil, 11361 Athens (GR); Ikonomopoulos, John, 152-34, Halandri (GR)
(74) Representative: Malamis, Alkisti-Irene

(57) **Abstract**

The invention investigates the association of the polymorphisms identified in the caprine (*Capra hircus*) *SlcI Ial* gene with innate resistance to *Mycobacterium avium* subsp. *paratuberculosis* (MAP). As a result, a test has been developed which determines susceptibility to paratuberculosis in a goat. The test is based on detection of the presence of particular microsatellite polymorphism (alleles) in the 3' UTR region of the gene. Certain microsatellites are associated with susceptibility to paratuberculosis.

## Description

### Field of the invention

The invention relates to determining susceptibility to paratuberculosis in a goat and therapy that is based on the SLC11A1 genotype present in the goat.

### Background to the invention

Paratuberculosis is a chronic granulomatous entiritis that affects mainly ruminants. The disease is caused by *Mycobacterium avium* subsp. *paratuberculosis* (*MAP*), an acid-fast bacterium with extreme resistance to the environment [9]. Paratuberculosis causes severe economic losses in infected herds and flocks [18]. Moreover, human exposure to *MAP* has been associated by many with the pathogenesis of Crohn's disease, a hypothesis that already consists the source of considerable concern [5, 7].

Control of paratuberculosis of ruminants usually relies on test and removal of the animals that can be characterized as positive by serology and cultivation or molecular detection of *MAP* in faecal samples [14]. Complete separation of the newborns from the adults until they become less sensitive to the infection, is usually a necessary addition to the above measure [16]. Vaccination is often proposed as a means to decrease the prevalence and the financial impact of the disease especially in enzootic animal populations [10]. However, this practice is regarded only as a last resort by the regulatory authorities, mainly because it doesn't prevent infection and at the same time it interferes with the measures applied for the control of tuberculosis [14].

Advances in immunology and genetics have permitted the identification and the study of genes that may be implicated in augmented resistance to infections. Recent scientific evidence indicate association of specific genetic polymorphisms with host-susceptibility/resistance to infections induced by intra-cellular parasites. Sensitivity of mice to infections induced by members of the *Leishmania* spp., *Mycobacterium* spp., and *Salmonella* spp., was found to be associated with specific genomic regions of chromosome 1 (*Ity, Lsh, Bcg*) that were later recognized as the *Nramp1* gene (Natural Resistance Associated Macrophage Protein), currently referred to as *Slc11a1* (solute carrier family 11 member 1) [6].

*SLC11A1* is expressed in macrophages and monocytes [4] and encodes a protein that is located on the phagolysosomic membrane where it functions as a divalent cation transporter (mostly for Fe²⁺ and Mg²⁺) [13]. The direction of transport is not yet well understood, although it seems likely that the cations move from the lumen of the phagolysosome to the cytosol thus preventing acquisition of these elements by intracellular parasites. However others suggest that this movement occurs in the opposite direction leading to an increased concentration of iron in the phagolysosome which favors the generation of oxygen intermediates and consequently bacterial killing through the Fenton reaction [12,19].

### Summary of the invention

The aim of the inventors' work was the sequence and structure analysis of the caprine (*Capra hircus*) *Slc11a1* gene and the investigation of the association of the polymorphisms identified in the latter, with innate resistance to *MAP.* The work led to the development of a test which determines susceptibility to paratuberculosis in a goat. The test is based on detection of the presence of particular microsatellite polymorphism (alleles) in the 3' UTR region of the gene (termed region A and region B herein). Certain microsatellites are associated with susceptibility to paratuberculosis, such as allele B₈.

Accordingly the invention provides a method for determining whether a goat is susceptible to paratuberculosis comprising typing region A and/or region B of the 3' UTR of the SLC11A1 gene of the goat to thereby determine susceptibility to paratuberculosis.

### Detailed description of the invention

The present invention relates to determining the genotype of the SLC11A1 gene to detect susceptibility to paratuberculosis in a goat (termed the 'subject' herein). The goat may be suspected of being at risk of disease. It is typically a newborn, for example less than 56 days, 28 days or 14 days old.

### Detection of microsatellite polymorphisms

The detection or genotyping of polymorphisms according to the invention may comprise contacting a polynucleotide of the subject with a specific binding agent for a polymorphism and determining whether the agent binds to the polynucleotide, wherein binding of the agent indicates the presence of the polymorphism, and lack of binding of the agent indicates the absence of the polymorphism.

The method is generally carried out in vitro on a sample from the subject. The sample typically comprises a body fluid and/or cells of the individual and may, for example, be obtained using a swab, such as a mouth swab. The sample may be a blood, urine, saliva, skin, cheek cell or hair root sample. The sample is typically processed before the method is carried out, for example DNA extraction may be carried out. The polynucleotide or protein in the sample may be cleaved either physically or chemically, for example using a suitable enzyme. In one embodiment the part of polynucleotide in the sample is copied or amplified, for example by cloning or using a PCR based method prior to detecting the polymorphism.

In the present invention, any one or more methods may comprise determining the presence or absence of one or more polymorphisms in the subject. The polymorphism is typically detected by directly determining the presence of the polymorphic sequence in a polynucleotide or protein of the subject. Such a polynucleotide is typically genomic DNA, mRNA or cDNA. The polymorphism may be detected by any suitable method such as those mentioned below.

A specific binding agent is an agent that binds with preferential or high affinity to the polynucleotide having the polymorphism but does not bind or binds with only low affinity to other polynucleotides. The specific binding agent may be a probe or primer. The probe may be a protein (such as an antibody) or an oligonucleotide. The probe may be labelled or may be capable of being labelled indirectly. The binding of the probe to the polynucleotide or protein may be used to immobilise either the probe or the polynucleotide or protein.

Generally in the method, determination of the binding of the agent to the polymorphism can be carried out by determining the binding of the agent to the polynucleotide of the subject. However in one embodiment the agent is also able to bind the corresponding wild-type sequence, for example by binding the nucleotides which flank the polymorphism position, although the manner of binding to the wild-type sequence will be detectably different to the binding of a polynucleotide containing the polymorphism.

The method may be based on an oligonucleotide ligation assay in which two oligonucleotide probes are used. These probes bind to adjacent areas on the polynucleotide which contains the polymorphism, allowing after binding the two probes to be ligated together by an appropriate ligase enzyme. However the presence of single mismatch within one of the probes may disrupt binding and ligation. Thus ligated probes will only occur with a polynucleotide that contains the polymorphism, and therefore the detection of the ligated product may be used to determine the presence of the polymorphism.

In one embodiment the probe is used in a heteroduplex analysis based system. In such a system when the probe is bound to polynucleotide sequence containing the polymorphism it forms a heteroduplex at the site where the polymorphism occurs and hence does not form a double strand structure. Such a heteroduplex structure can be detected by the use of single or double strand specific enzyme. Typically the probe is an RNA probe, the heteroduplex region is cleaved using RNAase H and the polymorphism is detected by detecting the cleavage products.

The method may be based on fluorescent chemical cleavage mismatch analysis which is described for example in PCR Methods and Applications 3, 268-71 (1994) and Proc. Natl. Acad. Sci. 85, 4397-4401 (1998).

In one embodiment a PCR primer is used that primes a PCR reaction only if it binds a polynucleotide containing the polymorphism, for example a sequence- or allele-specific PCR system, and the presence of the polymorphism may be determined by the detecting the PCR product. Preferably the region of the primer which is complementary to the polymorphism is at or near the 3' end of the primer. The presence of the polymorphism may be determined using a fluorescent dye and quenching agent-based PCR assay such as the Taqman PCR detection system. The specific binding agent may be capable of specifically binding the amino acid sequence encoded by a variant sequence. For example, the agent may be an antibody or antibody fragment. The detection method may be based on an ELISA system. The method may be an RFLP based system. This can be used if the presence of the polymorphism in the polynucleotide creates or destroys a restriction site that is recognised by a restriction enzyme.

The presence of the polymorphism may be determined based on the change which the presence of the polymorphism makes to the mobility of the polynucleotide or protein during gel electrophoresis. In the case of a polynucleotide single-stranded conformation polymorphism (SSCP) or denaturing gradient gel electrophoresis (DDGE) analysis may be used.

The presence of the polymorphism may be detected by means of fluorescence resonance energy transfer (FRET). In particular, the polymorphism may be detected by means of a dual hybridisation probe system. This method involves the use of two oligonucleotide probes that are located close to each other and that are complementary to an internal segment of a target polynucleotide of interest, where each of the two probes is labelled with a fluorophore. Any suitable fluorescent label or dye may be used as the fluorophore, such that the emission wavelength of the fluorophore on one probe (the donor) overlaps the excitation wavelength of the fluorophore on the second probe (the acceptor). A typical donor fluorophore is fluorescein (FAM), and typical acceptor fluorophores include Texas red, rhodamine, LC-640, LC-705 and cyanine 5 (Cy5).

In order for fluorescence resonance energy transfer to take place, the two fluorophores need to come into close proximity on hybridisation of both probes to the target. When the donor fluorophore is excited with an appropriate wavelength of light, the emission spectrum energy is transferred to the fluorophore on the acceptor probe resulting in its fluorescence. Therefore, detection of this wavelength of light, during excitation at the wavelength appropriate for the donor fluorophore, indicates hybridisation and close association of the fluorophores on the two probes. Each probe may be labelled with a fluorophore at one end such that the probe located upstream (5') is labelled at its 3' end, and the probe located downstream (3') is labelled at is 5' end. The gap between the two probes when bound to the target sequence may be from 1 to 20 nucleotides, preferably from 1 to 17 nucleotides, more preferably from 1 to 10 nucleotides, such as a gap of 1, 2, 4, 6, 8 or 10 nucleotides.

The first of the two probes may be designed to bind to a conserved sequence of the gene adjacent to a polymorphism and the second probe may be designed to bind to a region including one or more polymorphisms.

Polymorphisms within the sequence of the gene targeted by the second probe can be detected by measuring the change in melting temperature caused by the resulting base mismatches. The extent of the change in the melting temperature will be dependent on the number and base types involved in the nucleotide polymorphisms.

The polymorphic position may be typed directly, in other words by determining the nucleotide present at that position, or indirectly, for example by determining the nucleotide present at another polymorphic position that is in linkage disequilibrium with said polymorphic position. Polymorphisms which are in linkage disequilibrium with each other in a population are typically found together on the same chromosome. Typically one is found at least 30% of the times, for example at least 40 %, at least 50%, at least 70% or at least 90%, of the time the other is found on a particular chromosome in individuals in the population. Thus a polymorphism which is not a functional susceptibility polymorphism, but is in linkage disequilibrium with a functional polymorphism, may act as a marker indicating the presence of the functional polymorphism.

Polymorphisms which are in linkage disequilibrium with the polymorphisms mentioned herein are typically located within 500kb, preferably within 400kb, within 200kb, within 100kb, within 50kb, within 10kb, within 5kb, within 1 kb, within 500bp, within 100bp, within 50bp or within 10bp of the polymorphism.

A polynucleotide of the invention may be used as a primer, for example for PCR, or a probe. A polynucleotide or polypeptide of the invention may carry a revealing label. Suitable labels include radioisotopes such as 32P or 35S, fluorescent labels, enzyme labels or other protein labels such as biotin.

Polynucleotides of the invention may be used as a probe or primer which is capable of selectively binding to a polymorphism. The invention thus provides a probe or primer for use in a method according to the invention, which probe or primer is capable of selectively detecting the presence of a polymorphism. Preferably the probe is isolated or recombinant nucleic acid. The probe may be immobilised on an array, such as a polynucleotide array.

Such primers, probes and other fragments will preferably be at least 10, preferably at least 15 or at least 20, for example at least 25, at least 30 or at least 40 nucleotides in length. They will typically be up to 40, 50, 60, 70, 100 or 150 nucleotides in length. Probes and fragments can be longer than 150 nucleotides in length, for example up to 200, 300, 400, 500, 600, 700 nucleotides in length, or even up to a few nucleotides, such as five or ten nucleotides, short of a full length polynucleotide sequence of the invention.

The polynucleotides (e.g. primer and probes) of the invention may be present in an isolated or substantially purified form. They may be mixed with carriers or diluents which will not interfere with their intended use and still be regarded as substantially isolated. They may also be in a substantially purified form, in which case they will generally comprise at least 90%, e.g. at least 95%, 98% or 99%, of the polynucleotides or dry mass of the preparation.

In the method of the invention the presence or absence of the alleles mentioned in Table II may be detected by any suitable means. Typically in the method one or more of the polymorphisms listed in Table II is typed. Thus, the presence or absence of the polymorphism may be determined, typically in a polynucleotide from the subject, to ascertain whether or not the genome of the subject comprises the relevant polymorphism. In one embodiment, whether or not the genome of the subject comprises all of the polymorphisms listed in Table II is ascertained.

In a preferred embodiment, at least 1, 2, 3, 4, 5, 6, 7, 8, 9 or 10 of the polymorphisms shown in Table II are typed in the method of the invention. In one embodiment, a polymorphism which is in linkage to disequilibrium with a polymorphism shown in Table II is typed (in order to acesertain the presence of a polymorphism in Table II in the genome of the subject). In one embodiment, whether or not the polymorphisms which are typed are present on the same DNA strand is also determined.

In an alternative embodiment, polymorphisms are detected by sequencing the UTR region, or a part thereof, using a massively-parallel system. For example a sequencing-by-synthesis approach.

### Detection kit

The invention also provides a kit that comprises means for determining the presence or absence of one or more polymorphisms in a subject which define disease susceptibility of the subject. In particular, such means may include a specific binding agent, probe, primer, pair or combination of primers, or antibody, including an antibody fragment, as defined herein which is capable of detecting or aiding detection of a polymorphism. The primer or pair or combination of primers may be sequence specific primers which only cause PCR amplification of a polynucleotide sequence comprising the polymorphism to be detected, as discussed herein. The kit may also comprise a specific binding agent, probe, primer, pair or combination of primers, or antibody which is capable of detecting the absence of the polymorphism. The kit may further comprise buffers or aqueous solutions.

The kit may additionally comprise one or more other reagents or instruments which enable any of the embodiments of the method mentioned above to be carried out. Such reagents or instruments may include one or more of the following: a means to detect the binding of the agent to the polymorphism, a detectable label such as a fluorescent label, an enzyme able to act on a polynucleotide, typically a polymerase, restriction enzyme, ligase, RNAse H or an enzyme which can attach a label to a polynucleotide, suitable buffer(s) or aqueous solutions for enzyme reagents, PCR primers which bind to regions flanking the polymorphism as discussed herein, a positive and/or negative control, a gel electrophoresis apparatus, a means to isolate DNA from sample, a means to obtain a sample from the individual, such as swab or an instrument comprising a needle, or a support comprising wells on which detection reactions can be carried out. The kit may be, or include, an array such as a polynucleotide array comprising the specific binding agent, preferably a probe, of the invention. The kit typically includes a set of instructions for using the kit.

### Detection array

The invention also provides an array that comprises means for determining the presence or absence of one or more polymorphisms as defined herein. The array typically comprises probes or primers which are capable of selectively binding to the polymorphisms of the invention. The probes or primers are immobilised on a solid-surface such as a glass slide. Such primers or probes will preferably be at least 10, preferably at least 15 or at least 20, for example at least 25, at least 30 or at least 40 nucleotides in length. Typically an array will consist of 100, 1000, 10000, 100000, 1x10⁶ or 1x10⁷ different probes or primers.

Detection of the presence or absence of one or more polymorphisms in a subject using the array may be performed by any appropriate method. Typically such a method comprises taking a sample of genomic DNA from a subject, fragmenting the sample using, e.g. a restriction enzyme, and labelling the resulting fragments with a detectable marker such as a fluorescent label, which enables detection of the fragment when bound to the probes or primers of the array. The labelled fragments are incubated with the array under conditions suitable for hybridisation between the immobilised probes and the labelled fragments to occur. The genomic DNA sample may be amplified by any suitable non-specific amplification method prior to fragmenting.

### Screening for therapeutic agents

The present invention also relates to a method of screening for a therapeutic substance which treats paratuberculosis. In one embodiment, the method comprising contacting a candidate substance with a vector that comprises the sequence of any of the polymorphisms of the invention and determining whether the candidate substance is able to modulate expression from the vector. In a preferred embodiment, the vector comprises the sequence of any of the alleles shown in Table II.

The vector for use in the present invention group consists of bacterial vectors, virus vectors, nucleic acid based vectors and the like. The virus vectors include but are not limited to poxvirus, adenovirus, herpes virus, alphavirus, retrovirus, picornavirus, iridovirus and the like. The vector may express in a cell-free system, for example using commercially available *in vitro* transcription and translation systems. Alternatively the vector may be transfected into a non-human cell for expression. Transfection may be performed by any standard method, for example, electroporation, heat shock, or treatment with a suitable transfection reagent.

The vector may additionally comprise a marker to confirm expression. Suitable markers include antibiotic resistance genes, genes encoding fluorescent proteins or genes encoding reporter enzymes.

The method may be carried out *in vitro* or *in vivo.* In one embodiment the method is carried out on a non-human cell, cell culture or cell extract that comprises the polypeptide.

The method may also be carried out in vivo in a non-human subject which is transgenic for an allele as defined herein. The transgenic non-human subject is typically of a species commonly used in biomedical research and is preferably a laboratory strain. Suitable subjects include rodents, particularly a mouse, rat, guinea pig, ferret, gerbil or hamster. Most preferably the subject is a mouse.

Suitable candidate agents which may be tested in the above screening methods include antibody agents, for example monoclonal and polyclonal antibodies, single chain antibodies, chimeric antibodies and CDR-grafted antibodies. Furthermore, combinatorial libraries, defined chemical identities, peptide and peptide mimetics, oligonucleotides and natural agent libraries, such as display libraries may also be tested. The candidate agents may be chemical compounds, which are typically derived from synthesis around small molecules which may have any of the properties of the agent mentioned herein. Batches of the candidate agents may be used in an initial screen of, for example, ten substances per reaction, and the substances of batches which show modulation tested individually. The term 'agent' is intended to include a single substance and a combination of two, three or more substances. For example, the term agent may refer to a single peptide, a mixture of two or more peptides or a mixture of a peptide and a defined chemical entity.

The invention is illustrated by the following Examples:

### Examples

### 1. Overview of the present work

Breeding of animals with augmented resistance to paratuberculosis could greatly improve the outcome of control programs against the disease. Recent advances in immunology and genetics have permitted the identification of genes associated with the resistance to intracellular parasites. The role of *slc11a1* gene in the innate resistance to intracellular parasites has been documented in mice and is being investigated in many other animal species. In this study mRNA extracted from goat (*Capra hircus*) leucocytes was transcribed and the cDNA was used for the determination of the sequence of the *SLC11A1* gene. Analysis of the sequence revealed great homology of the coding sequence with its respective in sheep and cattle. Two polymorphic microsatellites containing a variable number of GT repeats were detected in the 3' UTR region. In order to examine the role of these polymorphisms in the innate resistance to paratuberculosis in goat the 3' UTR of the gene was sequenced in fifty adult animals randomly selected from a herd with known history of the disease. The infectious status of the animals was assessed by serology and Real Time PCR on faecal samples. The statistical analysis with Fisher's exact test revealed significant correlation between excretion of *Mycobacterium avium* subsp. *paratuberculosis* in faeces and genotype B₈ thus rendering carriers of this allele at a greater risk for the propagation of the bacterium in the environment.

### 2. MATERIALS AND METHODS

### 2.1 Sample plan

One sample of blood, serum and faeces was collected from each of 50 goats over the age of two, from a herd of 400 adult animals in Corinthia Province, Greece. The farm was selected on the grounds of established long-term record of paratuberculosis confirmed by clinical signs, antibody detection through ELISA and fecal culture (data not shown) and no previous record of tuberculin testing or paratuberculosis vaccination.
The animals included in the study, all of the native goat breed, corresponded to 25% of the adults at the time of sample collection (June 2007) and were selected randomly by ballot draw. Among those, some (7 out of 50) were recognized with clinical symptoms suggestive of active paratuberculosis such as poor body condition and non-responsive chronic diarrhea.
The samples collected were stored immediately in refrigerated containers and they were transported to the laboratory. The whole blood samples were used for DNA and RNA isolation that were incorporated to the sequence and structure analysis of the *Slc11a1* gene, whereas serum and faecal samples were used for ELISA and RT-PCR for the detection of *MAP* DNA and antibody, respectively.
DNA and RNA quantity and fragmentation were assessed by O.D. counts and electrophoresis on 2% agarose gel. With connection to the samples processed for detection of *MAP-*DNA, the presence of PCR inhibitors was assessed by a PCR assay targeted to an in-house gene (cytochrom C - data not shown).

### 2.2 DNA and RNA isolation

DNA isolation from faeces (50 samples) was performed with the Nucleospin tissue kit (Macherey Nagel, Düren, Germany). Briefly, 2g of faeces were homogenized with 20 ml of sterile water for 5 min. After 20 min of sedimentation at room temperature, 600 µl of the supernatant were transferred to a 1.5 ml tube containing 300 mg glass beads (Adiagene, Montpellier, France) and they were disrupted in a Mixer Mill (Retsch, Haan, Germany) at 30 Hz for 10 min. The liquid phase was collected and incubated overnight with 30µl of proteinase K (20 mg/ml) and then processed with the Nucleospin Tissue kit (Macherey Nagel, Düren, Germany) according to the manufacturer's instructions.

DNA isolation from blood (50 samples) was performed with the Nucleospin Blood kit (Macherey Nagel, Düren, Germany). RNA isolation was performed on 10 blood samples randomly selected among those collected from the herd with the RNA II kit (Macherey Nagel, Düren, Germany). Both were used according to the instructions of the manufacturer.

### 2.3 Sequence analysis of the caprine SLC11A1 gene

Sequence analysis of the caprine *SLC11A1* gene, relied on 10 of the blood samples that were collected. The RNA isolated from this material was used for the synthesis of first strand cDNA using SuperScript II reverse trascriptase (Invitrogen, Carlsbad, CA, USA). The cDNA-product was incorporated in PCR for the amplification of 4 overlapping gene fragments of approximately 500bp each (Table I). The sequence of the oligonucleotide primers was determined with the GeneRunner v. 3.05 (Hastings software Inc., Hastings, N.Y., USA) based on the *SLC11a1* gene sequence published for sheep (GenBank accession number U70255). Sequencing of the PCR products was performed using a DyeDeoxy Terminator Cycle Sequencing in an automated ABI Prism 373 DNA sequencer (Applied Biosystems, Foster City, CA, USA). Sequencing data ware analyzed with the SeqEd 1.0.3 software (Applied biosystems, Foster City, CA, USA) and assembled using GeneRunner v. 3.05.

### 2.4 Structure analysis of 3' UTR polymorphisms of the caprine SLC11A1 gene.

The structure analysis of the 3' UTR end of the caprine *SLC11A1* gene was performed on genomic DNA isolated from the whole blood samples, collected from the 50 goats that were included in this study. The procedure was targeted to the part of 3' UTR that includes the two polymorphic microsatellites revealed by the sequence analysis of the *SLC11A1* gene in goat. The latter data was also used to determine the sequence of the oligonucleotide primers incorporated to the relevant PCR assay (Table I). Sequence analysis of the PCR products was performed with DyeDeoxy Terminator Cycle Sequencing in an automated ABI Prism 373 DNA sequencer (Applied Biosystems, Foster City, CA, USA) using the same primers as in PCR. The whole procedure was repeated for samples producing low quality data.

### 2.5 Detection of MAP-specific antibodies by ELISA.

Detection of *MAP*-specific antibodies was performed on our serum samples collected by a commercially available ELISA according to the instructions of the manufacturer (Pourquier, Montpellier, France). The method incorporates a protoplasmic extract of *MAP* and includes a a pre-incubation of samples with an extract of *Mycobacterium phlei* in order to minimize cross-reaction with atypical mycobacteria.

### 2.6 Detection of MAP DNA in faeces by RT-PCR

Detection of *MAP* DNA was performed as previously described on the 50 faecal samples collected from the animal population included in this study [15]. Sample testing was performed in a LightCycler 2 instrument (Roche, Basel, Switzerland). Positive and negative controls incorporated in each assay corresponded to approximately 10% of the samples tested per batch and consisted respectively of the reaction mixture containing *MAP* DNA (positive control) and total DNA of confirmed PCR-negative faecal samples or water (negative control).

### 2.7 Statistical analysis.

The investigation of the association between genetic polymorphisms in the 3' UTR of the caprine *SLC11A1* gene and the presence of *MAP* specific antibodies in serum and/or *MAP* DNA in faeces was determined by the Fisher's exact test using the Graphpad Instat software, version 3.05 (Graphpad software Inc., California).

### 3. RESULTS

### 3.1 Sequence analysis of the SLC11A1 gene in goat.

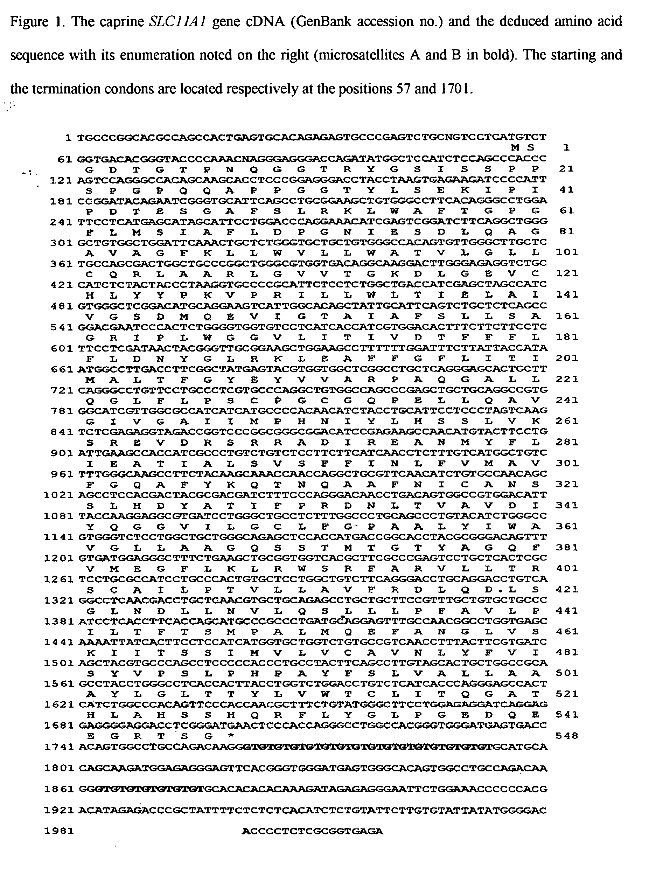

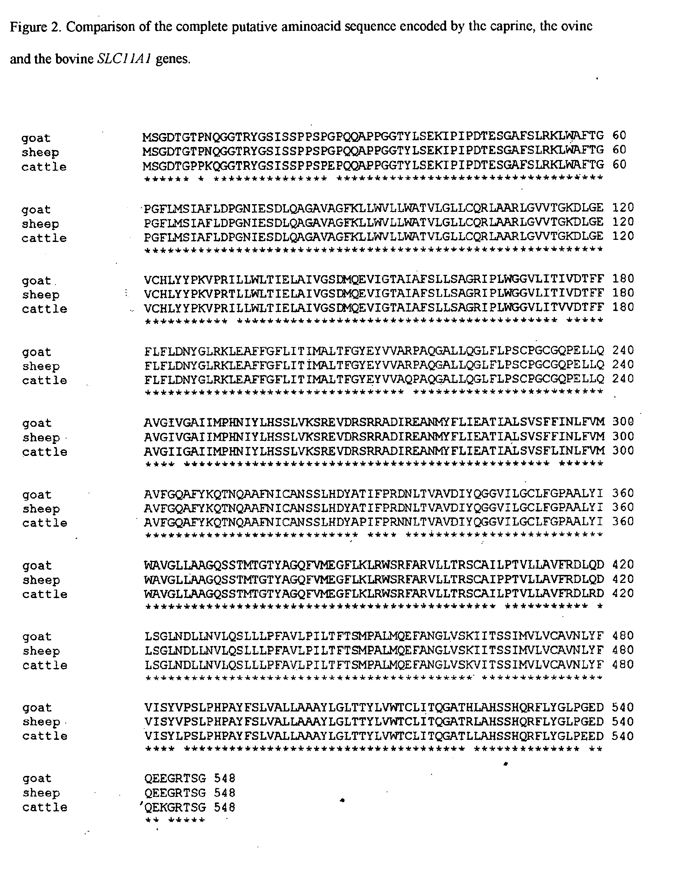

The complete goat *SLC11A1* gene coding sequence is shown in Figure 1. The assembled cDNA was 2004 bases long and presented a 1648 bases Open Reading Frame (ORF) encoding a 548 residue protein with calculated molecular weight 59.4 KDa. The ORF shows 99% and 97% identity with the relevant ovine (U70255) and bovine (U12862) genes respectively. The same percentages apply with connection to the similarity of the putative goat protein with that of sheep and bovines (Figure 2).

### 3.2 Structure analysis of the polymorphic alleles in the 3' UTR.

The sequence of the 3' UTR end of the caprine *SLC11A1* gene was determined in 49 out of the 50 animals tested, as 1 sample failed to produce nucleotide sequence data of adequate quality and was thereby rejected. Structure analysis of the 3' UTR end of goat *SLC11A1* gene revealed 2 microsatellites consisting of a variable number of guanine-cytosine (GT) repeats named region A and B respectively (Figure 1). Microsatellites A and B are found respectively at 61 and 162 bases downstream from the end codon. Microsatellite A consists of 14-18 GT repeats while microsatellite B consists of 7-8 GT repeats (Tables II and III).

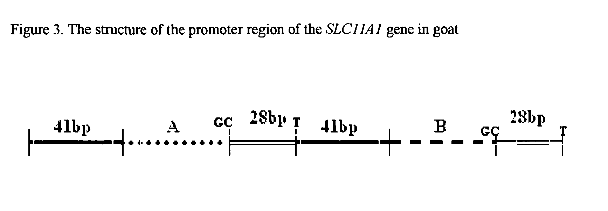

Microsatellites A and B are delimited upstream by an identical sequence of 41 nucleotides and downstream by a 28 nucleotide sequence with high homology between the two microsatellites. The end of the first 28 nucleotides sequences signs the beginning of the second 41 nucleotide sequence (Figure 3)

### 3.3 Detection of MAP-specific DNA and antibodies.

*MAP*-specific antibodies were detected by ELISA in 18 animals (36%, 18 out of 50) with 2 (4%, 2 aou of 50) producing doubtful results that were excluded from the statistical evaluation. RT-PCR performed on faeces produced positive results in the 16 of the tested animals (32%, 16 out of 50).

### 3.4 Statistical analysis.

Statistical analysis for the investigation of association between detection of *MAP* DNA and antibody, and the polymorphisms of the 3' UTR end of the caprine *SLC11A1* gene produced significant results for the B₈ allele of the polymorphic region B, and the presence of *MAP* DNA in faeces (p=0.0157) (table V). The Fisher's exact test did not provide evidence of statistical significance between ELISA-positivity and any allele of the polymorphic regions A (table IV) and B (table V). Similarly there was no association between RT-PCR positivity in faeces and the alleles recorded in region A (table IV).

### 4. DISCUSSION

The sequence of the complete caprine *SLC11A1* gene is presented here for the first time, together with a structure analysis of its 3' UTR end. As expected, the level of similarity of the studied gene with its ovine and bovine homologue, proved high. The same was recorded with connection to the relevant proteom that exhibits only minor aminoacid differences among the three animal species, which shouldn't influence significantly the conformation and the functionality of the putative protein, as they seem to be placed in non critical positions.

However structure analysis of the 3' UTR end of the *SLC11A1* gene revealed specific characteristics that have not been reported with connection to the ovine homologue. In more detail, the 3' UTR end of the caprine *SLC11A1* gene harbors two polymorphic microsatellites as opposed to sheep that possess one [17]. The structure of the specific genomic region in goats seems therefore more similar to that of bovines that also host two microsatellites, albeit only one has been studied [1]. Notably it is well documented that goats develop paratuberculosis in a form that resembles more that of bovines than sheep that seem to be less sensitive to the infection manifesting milder symptoms with more chances of autosterilization (Clark, 1997). The presence of these microsatellites could be attributed to a duplication of the relevant DNA fragment that specifically for goats it also includes one segment of 41 bases upstream and another one of 28 bases downstream. This hypothesis is supported by the fact that these segments are found in continuity.

Considering previous reports on the association of the polymorphisms of the 3' UTR end of the *SLC11A1* gene with innate resistance of various animal species to microbial infections induced by intracellular pathogens, this study extended to a similar investigation at a preliminary level, with connection to goats and *MAP-*infection, for which to the best of our knowledge this is the first report. The association of the polymorphisms recorded here was assessed with connection to seropositivity to *MAP,* and detection of *MAP-*DNA in faecal samples by RT-PCR. Both parameters were used as indicative of the infection status of the targeted animal population that was considered exposed to *MAP* since it consisted of only adult goats that were all reared semi-extensively (sharing posture, bedding and space) for at least two years, in a farm with a long record of paratuberculosis and active cases at the time of sample collection. Furthermore the herd under study, had to have no history of paratuberculosis vaccination or tuberculin testing, which could theoretically generate false positive result by the ELISA incorporated in the evaluation of the animals.

Based on the statistical analysis of our results, the A₁₆ genotype observed in the polymorphic region A proved very common among the animals tested (91.84%) and was therefore disqualified from further evaluation. However, B₈ allele of the region B was found to be associated at a statistically significant level with RT-PCR detectable *MAP*-DNA in the faeces. Obviously this finding does not imply a functional significance of the specific polymorphism that may consist just a genetic marker for the phenotype mentioned above. If a functional role of the B₈ allele of the region B was hypothesized, it would have to be assessed in connection with the altered expression of the *SLC11A1* gene caused by the elongation of its promoter or the interaction of this polymorphism with the binding site of a transcriptional factor. The altered expression of the gene could lead to decreased protein synthesis and thus to the reduction of the concentration of the ion-pumps associated *SLC11A1* gene-activity, and consequently to the alteration of the intraphagolysosomal environment that could favor the survival of the parasite, its multiplication and finally its excretion with faeces.

Infection with *MAP* activates a cell-mediated response with a fair protective effect, able to impede to some extend the spread of the parasite while humoral response develops years after the infection, and has been linked with decreased ability of the host to deal with the parasite [8]. These characteristics of the immune reaction triggered by *MAP*-infection may account for the results recorded here including the lack of statistically significant association of the ELISA-positive results with the genetic polymorphisms detected. Capparelli et al. reported association of the BB genotype with resistance to *Brucella* spp., in the water buffalo (*Bubalus bubalis*), and higher basal expression level of the *SLC11A1* gene. In support of this association, macrophages collected from the same animals exhibited increased production of radical oxygen and nitrate intermediates after their in-vitro exposure to *Brucella* spp. Similarly, increased sensitivity to paratuberculosis was reported for sheep of the 162 genotype that was found to be associated at a statistically significant level with the development of the severe form of the disease [Reddacliff et al 2005]. Association of the B₈ allele of the region B with fecal excretion of *MAP* recorded in goats carrying the specific genetic polymorphism to paratuberculosis, suggests that a larger-scale study may prove very significant with connection to the investigation of innate resistance/sensitivity of goats to paratuberculosis. Indeed, susceptible animals contribute to the survival of *MAP* and its excretion in the environment thus rendering the application of control measures for the disease a very difficult task. The maintenance of *MAP* in the environment increases human exposure to the bacterium with potentially significant public health implications. The selection of animals genetically resistant to paratuberculosis or the removal of those with a tendency to sustain the infection may prove a vital asset for the eradication of the disease.

### REFERENCES

[1] Ables G.P., Nishibori M., Kanemaki M., Watanabe T., Sequence analysis of the NRAMP1 genes from different bovine and buffalo breeds, J. Vet. Med. Sci. (2002) 64:1081-1083.
[2] Blackwell M.J., Braton H., White K.J., Searle S., Baker A., Williams H., Shaw M., Genomic organization and sequence of the human NRAMP gene: Identification and mapping of a promoter polymorphism, Mol. Med. 1:194-205.
[3] Bussmann V., Lantier I., Pitel F., Patri S., Nau F., Gros P., Elsen J.M., Lantier F., cDNA cloning, structural organization, and expression of the sheep NRAMP1 gene, Mamm. Genome. (1998) 9:1027-1031.
[4] Canonne-Hergaux F., Gruenheid S., Govoni G., Gros P., The Nramp1 protein and its role in resistance to infection and macrophage function, Proc. Assoc. Am. Physicians (1999) 111:283-1289.
[5] Chacon O., Bermudez L.E., Barletta_R.G., Johne's disease, inflammatory bowel disease, and Mycobacterium paratuberculosis, Annu. Rev. Microbiol. (2004) 58:329-63.
[6] Chermesh I., Azriel A., Alter-Koltunoff M., Eliakim R., Karban A., Levi B.Z., Crohn's disease and SLC11A1 promoter polymorphism, Dig. Dis. Sci. (2007) 52:1632-1635.
[7] Chiodini R.J., Van Kruiningen H.J., Merkal R.S., Thayer W.R. Jr., Coutu J.A., Characteristics of an unclassified Mycobacterium species isolated from patients with Crohn's disease, J. Clin. Microbiol. (1984) 20:966-971.
[8] Clarke C.J., The pathology and pathogenesis of paratuberculosis in ruminants and other species, J. Comp. Pathol. (1997) 116:217-261.
[9] Collins M.T, Spaihr U., Murphy P.M., Ecological characteristics of Mycobacterium paratuberculosis, Bull. Of the IDF (2001) 362:32-40.
[10] Corpa J.M., Pérez V., Sánchez M.A., Marín J.F., Control of paratuberculosis (Johne's disease) in goats by vaccination of adult animals, Vet. Rec. (2000) 146:195-196.
[11] Feng J., Li Y., Hashad M., Schurr E., Gros P., Adams L.G., Templeton J.W., Bovine natural resistance associated macrophage protein 1 (Nramp1) gene, Genome Res. (1996) 10:956-964.
[12] Forbes J.R., Gros P., Iron, manganese, and cobalt transport by Nramp1 (Slc11a1) and Nramp2 (Slc11a2) expressed at the plasma membrane, Blood (2003) 102:1884-1892.
[13] Gruenheid S., Pinner E., Desjardins M., Gros P., Natural resistance to infection with intracellular pathogens: the Nramp1 protein is recruited to the membrane of the phagosome, J. Exp. Med. (1997) 185:717-730.
[14] Kennedy D.J., Benedictus G., Control of Mycobacterium avium subsp. paratuberculosis infection in agricultural species. Rev. Sci .Tech. (2001) 20:151-179.
[15] Kim S.G., Kim E.H., Lafferty C.J., Miller L.J., Koo H.J., Stehman S.M., Shin S.J., Use of conventional and real-time polymerase chain reaction for confirmation of Mycobacterium avium subsp. paratuberculosis in a broth-based culture system ESP II, J. Vet. Diagn. Invest. (2004) 16:448-453.
[16] Manning E.J., Collins M.T., Mycobacterium avium subsp. paratuberculosis: pathogen, pathogenesis and diagnosis, Rev. Sci. Tech. (2001) 20:133-150.
[17] Matthews G.D., Crawford A.M., Cloning, sequencing and linkage mapping of the RAMP1 gene of sheep and deer, Anim. Genet. (1998) 29:1-6.
[18] Ott S.L., Wells S.J., Wagner B.A., Herd-level economic losses associated with Johne's disease on US dairy operations, Prev. Vet. Med (1999) 40:179-192.
[19] Paixão T.A., Poester F.P., Carvalho Neta A.V., Borges A.M., Lage A.P., Santos R.L., NRAMP1 3' untranslated region polymorphisms are not associated with natural resistance to Brucella abortus in cattle, Infect. Immun. (2007) 75:2493-2499.

**Table I. Description of the PCR assays incorporated for the sequence analysis of the caprine SLC11A1 gene (No: 1), and its 3' UTR end (No: 2).**

| No | Primer code | Primer sequence* Orientation 5'→3' | Position^{**} | Tm (°C) | Temperature profile |
|---|---|---|---|---|---|
| 1 | Pair 1 | CTTGCCATGCCAGTGAG | 17-34 | 56 | |
| | | TCGATGGTCAGCCAGAGGA | 490-508 | 56 | |
| | Pair 2 | GTGGTGACAGGCAAGGACTT | 426-445 | 56 | 95°C X 5 min |
| | | ATGTTGGCTTCTCGGATGTC | 909-928 | 56 | 35 cycles of 94° X 45 sec, annealing at Tm of |
| | Pair 3 | GGACATCCGAGAAGCCAAC | 908-926 | 56 | each primer pair for 50 sec, 72°C X 60 sec |
| | | CAAACGGAAGCAGCAGGC | 1390-1407 | 56 | 72°C X 8 min |
| | Pair 4 | CACTGTGCTCCTGGCTGTCTTC | 1316-1337 | 61 | |
| | | CAGGCCACTGTGGTCACTCATC | 1768-1789 | 61 | |
| 2 | NrampF | ACCTGGTCTGGACCTGTCTCATCA | 1621-1644 | 56 | 95°C X 5min 35 cycles of 95°C X 40 sec, 56°C X 40 sec, |
| | NrampR | CATTGCAAGGTAGGTGTCCCCAT | 1944-1966 | 56 | 72°C X 45 sec 72°C X 8 min |

| | | | | | |
|---|---|---|---|---|---|
| * 50 µl volume/reaction with 1X PCR buffer (provided with the DNA polymerase), 2mM MgCl₂, 10 pmol of each primer, 200µM of each dNTP, 1.25 U of Taq DNA polymerase (Taq DNA polymerase, Qiagen, Germany) and 5 µl of cDNA solution in a ** Position refers to the published sequence of the ovine *SLC11A1* gene (U70255) | | | | | |

**Table II. Sequence of the polymorphic alleles detected in the 3' UTR end of the SLC11A1 gene in goat.**

| Name | Sequence |
|---|---|
| A₁₄B₇ | AAGG(GT)₁₄GCATGC... AAGG(GT)₇GCACAC |
| A₁₅B₈ | AAGG(GT)₁₅GCATGC... AAGG(GT)₈GCACAC |
| A₁₆B₇ | AAGG(GT)₁₆GCATGC... AAGG(GT)₇GCACAC |
| A₁₆B₈ | AAGG(GT)₁₆GCATGC... AAGG(GT)₈GCACAC |
| A₁₈B₈ | AAGH(GT)₁₈GCATGC... AAGG(GT)₈GCACAC |

**Table III. Frequency of the polymorphic alleles identified in microsatellites A and B of the 3' UTR end of the goat SLC11A1 gene.**

| Frequency of polymorphic alleles | | | | |
|---|---|---|---|---|
| (%) | | | Genotype | Frequency |
| Number of GT repeats | Region A | Region B | | (%) |
| 7 | - | 24 (49.98) | A₁₄B₇ | 2 (4.08) |
| 8 | - | 25 (51.02) | A₁₅B₈ | 1 (2.04) |
| 14 | 2 (4.08) | - | A₁₆B₇ | 23 (46.94) |
| 15 | 1 (2.04) | - | A₁₆B₈ | 22 (44.90) |
| 16 | 45 (91.84) | - | A₁₈B₈ | 1 (2.04) |
| 18 | 1 (2.04) | - | | |

**Table IV. Fisher's exact test on the frequency of the GT repeats detected in the regions A and B of the promoter of the caprine SLC11A1 gene with connection to the ELISA and PCR results recorded from the same animals (Statistical significance is considered for p<0.05).**

| | Number of GT repeats | ELISA | | PCR feaces | |
|---|---|---|---|---|---|
| | | Positive | Negative | Positive | Negative |
| Region A | 14 | 0 | 2 | 0 | 2 |
| | 15 | 0 | 1 | 0 | 1 |
| | 16 | 17 | 26 | 16 | 29 |
| | 18 | 1 | 0 | 0 | 1 |
| | | p=0.3206 | | p=0.1444 | |
| Region B | 7 | 7 | 18 | 4 | 21 |
| | 8 | 11 | 11 | 12 | 12 |
| | | p=0.5495 | | p=0.0157 | |

## Claims

1. A method for determining whether a goat is susceptible to paratuberculosis comprising typing region A and/or region B of the 3' UTR of the SLC11A1 gene of the goat to thereby determine susceptibility to paratuberculosis.

2. A method according to claim 1 comprising determining whether the B8 allele of region B is present in the goat.

3. A method according to claim 1 or 2 wherein the presence or absence of any of the region A alleles and/or region B alleles shown in Table II is determined.

4. A method according to any one of the preceding claims wherein both of the chromosomes of the goat are typed.

5. A method according to claim any one of the preceding claims wherein the goat is a newborn, preferably less than 28 days old.

6. A non-human cell comprising a polynucleotide that comprises any of alleles for region A and/or region B shown in Table II.

7. A kit for carrying out the method of any one of claims 1 to 5 comprising a means, optionally polynucleotides, for detecting one or more of alleles of region A and/or region B, and optionally for detecting one or more of the regions shown in Table II.

8. A kit according to claim 7 comprising at least one polynucleotide probe or primer which is capable of detecting one or more of alleles of region A and/or region B, and optionally capable of detecting one or more of the regions shown in Table II.

9. A polynucleotide array for determining the susceptibility of a goat to paratuberculosis, comprising a means for detecting one or more region A and/or region B alleles of the goat SLC11A1 gene, wherein the array optionally comprises at least one polynucleotide probe or primer which is capable of detecting at least region A and/or region B shown in Table II.

10. A method of screening for a therapeutic substance which treats paratuberculosis in goats comprising contacting a candidate substance with a vector that that comprises the sequence of any of the region A or region B alleles shown in Table II and determining whether the candidate substance is able to modulate expression from the vector.

11. A therapeutic substance which prevents or treats paratuberculosis for use in a method of treatment comprising determining whether a goat is susceptible to paratuberculosis by a method according to any one of claims 1 to 5, and if the individual is determined to be susceptible comprising administering the therapeutic substance to the individual, wherein the therapeutic substance is optionally a vaccine against paratuberculosis.


SEQUENCE LISTING

<110>  IKONOMOPOULOS, John

<120>  PARATUBERCULOSIS SUSCEPTIBILITY TEST

<130>  epo/nanomy/goat

<140>  EP 09003292.1
<141>  2009-03-06

<160>  24

<170>  PatentIn version 3.5

<210>  1
<211>  18
<212>  DNA
<213>  Artificial Sequence

<220>
<223>  forward primer pair 1

<400>  1
gcttgccatg ccagtgag                                                    18


<210>  2
<211>  19
<212>  DNA
<213>  Artificial Sequence

<220>
<223>  reverse primer pair 1

<400>  2
tcgatggtca gccagagga                                                   19


<210>  3
<211>  20
<212>  DNA
<213>  Artificial Sequence

<220>
<223>  forward primer pair 2

<400>  3
gtggtgacag gcaaggactt                                                  20


<210>  4
<211>  20
<212>  DNA
<213>  Artificial Sequence

<220>
<223>  reverse primer pair 2

<400>  4
atgttggctt ctcggatgtc                                                  20


<210>  5
<211>  19
<212>  DNA
<213>  Artificial Sequence

<220>
<223>  forward primer pari 3

<400>  5
ggacatccga gaagccaac                                                    19


<210>  6
<211>  18
<212>  DNA
<213>  Artificial Sequence

<220>
<223>  reverse primer pair 3

<400>  6
caaacggaag cagcaggc                                                     18


<210>  7
<211>  22
<212>  DNA
<213>  Artificial Sequence

<220>
<223>  forward primer pair 4

<400>  7
cactgtgctc ctggctgtct tc                                                22


<210>  8
<211>  22
<212>  DNA
<213>  Artificial Sequence

<220>
<223>  reverse primer pair 4

<400>  8
caggccactg tggtcactca tc                                                22


<210>  9
<211>  24
<212>  DNA
<213>  Artificial Sequence

<220>
<223>  NrampF primer

<400>  9
acctggtctg gacctgtctc atca                                              24


<210>  10
<211>  23
<212>  DNA
<213>  Artificial Sequence

<220>
<223>  NrampR primer

<400>  10
cattgcaagg taggtgtccc cat                                               23


<210>  11

<211>  38
<212>  DNA
<213>  Capra hircus

<220>
<221>  misc_feature
<223>  SLC11A1 allele A14B7

<400>  11
aagggtgtgt gtgtgtgtgt gtgtgtgtgt gtgcatgc                                38

<210>  12
<211>  24
<212>  DNA
<213>  Capra hircus

<220>
<221>  misc_feature
<223>  SLC11A1 allele A14B7

<400>  12
aagggtgtgt gtgtgtgtgc acac                                               24

<210>  13
<211>  40
<212>  DNA
<213>  Capra hircus

<220>
<221>  misc_feature
<223>  SLC11A1 allele A15B8

<400>  13
aagggtgtgt gtgtgtgtgt gtgtgtgtgt gtgtgcatgc                              40

<210>  14
<211>  26
<212>  DNA
<213>  Capra hircus

<220>
<221>  misc_feature
<223>  SLC11A1 allele A15B8

<400>  14
aagggtgtgt gtgtgtgtgt gcacac                                             26

<210>  15
<211>  42
<212>  DNA
<213>  Capra hircus

<220>
<221>  misc_feature
<223>  SLC11A1 allele A16B7

<400>  15
aagggtgtgt gtgtgtgtgt gtgtgtgtgt gtgtgtgcat gc                           42

<210>  16
<211>  24
<212>  DNA
<213>  Capra hircus

<220>
<221>  misc_feature
<223>  SLC11A1 allele A16B7

<400>  16
aagggtgtgt gtgtgtgtgc acac                                              24

<210>  17
<211>  42
<212>  DNA
<213>  Capra hircus

<220>
<221>  misc_feature
<223>  SLC11A1 allele A16B8

<400>  17
aagggtgtgt gtgtgtgtgt gtgtgtgtgt gtgtgtgcat gc                          42

<210>  18
<211>  26
<212>  DNA
<213>  Capra hircus

<220>
<221>  misc_feature
<223>  SLC11A1 allele A16B8

<400>  18
aagggtgtgt gtgtgtgtgt gcacac                                            26

<210>  19
<211>  46
<212>  DNA
<213>  Capra hircus

<220>
<221>  misc_feature
<223>  SLC11A1 allele A18B8

<400>  19
aagggtgtgt gtgtgtgtgt gtgtgtgtgt gtgtgtgtgt gcatgc                      46

<210>  20
<211>  26
<212>  DNA
<213>  Capra hircus

<220>
<221>  misc_feature
<223>  SLC11A1 allele A18B8

<400>  20

aagggtgtgt gtgtgtgtgt gcacac 26

<210> 21
<211> 1998
<212> DNA
<213> Capra hircus

<220>
<221> misc_feature
<222> (48)..(48)
<223> n is a, c, g, or t

<220>
<221> CDS
<222> (55)..(1701)

<220>
<221> misc_feature
<222> (82)..(82)
<223> n is a, c, g, or t

<400> 21
tgcccggcac gccagccact gagtgcacag agagtgcccg agtctgcngt cctc atg 57
Met
1

tct ggt gac acg ggt acc cca aac nag gga ggg acc aga tat ggc tcc 105
Ser Gly Asp Thr Gly Thr Pro Asn Gln Gly Gly Thr Arg Tyr Gly Ser
            5                   10                  15

atc tcc agc cca ccc agt cca ggg cca cag caa gca cct ccc gga ggg 153
Ile Ser Ser Pro Pro Ser Pro Gly Pro Gln Gln Ala Pro Pro Gly Gly
        20                  25                  30

acc tac cta agt gag aag atc ccc att ccg gat aca gaa tcg ggt gca 201
Thr Tyr Leu Ser Glu Lys Ile Pro Ile Pro Asp Thr Glu Ser Gly Ala
    35                  40                  45

ttc agc ctg cgg aag ctg tgg gcc ttc aca ggg cct gga ttc ctc atg 249
Phe Ser Leu Arg Lys Leu Trp Ala Phe Thr Gly Pro Gly Phe Leu Met
50                  55                  60                  65

agc ata gca ttc ctg gac cca gga aac atc gag tcg gat ctt cag gct 297
Ser Ile Ala Phe Leu Asp Pro Gly Asn Ile Glu Ser Asp Leu Gln Ala
                70                  75                  80

ggg gct gtg gct gga ttc aaa ctg ctc tgg gtg ctg ctg tgg gcc aca 345
Gly Ala Val Ala Gly Phe Lys Leu Leu Trp Val Leu Leu Trp Ala Thr
            85                  90                  95

gtg ttg ggc ttg ctc tgc cag cga ctg gct gcc cgg ctg ggc gtg gtg 393
Val Leu Gly Leu Leu Cys Gln Arg Leu Ala Ala Arg Leu Gly Val Val
        100                 105                 110

aca ggc aag gac ttg gga gag gtc tgc cat ctc tac tac cct aag gtg 441
Thr Gly Lys Asp Leu Gly Glu Val Cys His Leu Tyr Tyr Pro Lys Val
    115                 120                 125

ccc cgc att ctc ctc tgg ctg acc atc gag cta gcc atc gtg ggc tcg 489
Pro Arg Ile Leu Leu Trp Leu Thr Ile Glu Leu Ala Ile Val Gly Ser
130                 135                 140                 145

gac atg cag gaa gtc att ggc aca gct att gca ttc agt ctg ctc tca 537
Asp Met Gln Glu Val Ile Gly Thr Ala Ile Ala Phe Ser Leu Leu Ser
                150                 155                 160

gcc gga cga atc cca ctc tgg ggt ggt gtc ctc atc acc atc gtg gac 585
Ala Gly Arg Ile Pro Leu Trp Gly Gly Val Leu Ile Thr Ile Val Asp
            165                 170                 175

act ttc ttc ttc ctc ttc ctc gat aac tac ggg ttg cgg aag ctg gaa 633
Thr Phe Phe Phe Leu Phe Leu Asp Asn Tyr Gly Leu Arg Lys Leu Glu
        180                 185                 190

gcc ttt ttt gga ttt ctt att acc ata atg gcc ttg acc ttc ggc tat 681
Ala Phe Phe Gly Phe Leu Ile Thr Ile Met Ala Leu Thr Phe Gly Tyr
    195                 200                 205

gag tac gtg gtg gct cgg cct gct cag gga gca ctg ctt cag ggc ctg 729
Glu Tyr Val Val Ala Arg Pro Ala Gln Gly Ala Leu Leu Gln Gly Leu
210                 215                 220                 225

ttc ctg ccc tcg tgc cca ggc tgt ggc cag ccc gag ctg ctg cag gcc 777
Phe Leu Pro Ser Cys Pro Gly Cys Gly Gln Pro Glu Leu Leu Gln Ala
                230                 235                 240

gtg ggc atc gtt ggc gcc atc atc atg ccc cac aac atc tac ctg cat 825
Val Gly Ile Val Gly Ala Ile Ile Met Pro His Asn Ile Tyr Leu His
            245                 250                 255

tcc tcc cta gtc aag tct cga gag gta gac cgg tcc cgg cgg gcg gac 873
Ser Ser Leu Val Lys Ser Arg Glu Val Asp Arg Ser Arg Arg Ala Asp
        260                 265                 270

atc cga gaa gcc aac atg tac ttc ctg att gaa gcc acc atc gcc ctg 921
Ile Arg Glu Ala Asn Met Tyr Phe Leu Ile Glu Ala Thr Ile Ala Leu
    275                 280                 285

tct gtc tcc ttc ttc atc aac ctc ttt gtc atg gct gtc ttt ggg caa 969
Ser Val Ser Phe Phe Ile Asn Leu Phe Val Met Ala Val Phe Gly Gln
290                 295                 300                 305

gcc ttc tac aag caa acc aac cag gct gcg ttc aac atc tgt gcc aac 1017
Ala Phe Tyr Lys Gln Thr Asn Gln Ala Ala Phe Asn Ile Cys Ala Asn
                310                 315                 320

agc agc ctc cac gac tac gcg acg atc ttt ccc agg gac aac ctg aca 1065
Ser Ser Leu His Asp Tyr Ala Thr Ile Phe Pro Arg Asp Asn Leu Thr
            325                 330                 335

gtg gcc gtg gac att tac caa gga ggc gtg atc ctg ggc tgc ctc ttt 1113
Val Ala Val Asp Ile Tyr Gln Gly Gly Val Ile Leu Gly Cys Leu Phe
        340                 345                 350

ggc cct gca gcc ctg tac atc tgg gcc gtg ggt ctc ctg gct gct ggg 1161
Gly Pro Ala Ala Leu Tyr Ile Trp Ala Val Gly Leu Leu Ala Ala Gly
    355                 360                 365

cag agc tcc acc atg acc ggc acc tac gcg gga cag ttt gtg atg gag 1209
Gln Ser Ser Thr Met Thr Gly Thr Tyr Ala Gly Gln Phe Val Met Glu
370                 375                 380                 385

ggc ttt ctg aag ctg cgg tgg tca cgc ttc gcc cga gtc ctg ctc act 1257
Gly Phe Leu Lys Leu Arg Trp Ser Arg Phe Ala Arg Val Leu Leu Thr
                390                 395                 400

cgc tcc tgc gcc atc ctg ccc act gtg ctc ctg gct gtc ttc agg gac 1305
Arg Ser Cys Ala Ile Leu Pro Thr Val Leu Leu Ala Val Phe Arg Asp
            405                 410                 415

ctg cag gac ctg tca ggc ctc aac gac ctg ctc aac gtg ctg cag agc 1353
Leu Gln Asp Leu Ser Gly Leu Asn Asp Leu Leu Asn Val Leu Gln Ser
        420                 425                 430

ctg ctg ctt ccg ttt gct gtg ctg ccc atc ctc acc ttc acc agc atg 1401
Leu Leu Leu Pro Phe Ala Val Leu Pro Ile Leu Thr Phe Thr Ser Met
    435                 440                 445

ccc gcc ctg atg cag gag ttt gcc aac ggc ctg gtg agc aaa att atc 1449
Pro Ala Leu Met Gln Glu Phe Ala Asn Gly Leu Val Ser Lys Ile Ile
450                 455                 460                 465

act tcc tcc atc atg gtg ctg gtc tgt gcc gtc aac ctt tac ttc gtg 1497
Thr Ser Ser Ile Met Val Leu Val Cys Ala Val Asn Leu Tyr Phe Val
                470                 475                 480

atc agc tac gtg ccc agc ctc ccc cac cct gcc tac ttc agc ctt gta 1545
Ile Ser Tyr Val Pro Ser Leu Pro His Pro Ala Tyr Phe Ser Leu Val
            485                 490                 495

gca ctg ctg gcc gca gcc tac ctg ggc ctc acc act tac ctg gtc tgg 1593
Ala Leu Leu Ala Ala Ala Tyr Leu Gly Leu Thr Thr Tyr Leu Val Trp
        500                 505                 510

acc tgt ctc atc acc cag gga gcc act cat ctg gcc cac agt tcc cac 1641
Thr Cys Leu Ile Thr Gln Gly Ala Thr His Leu Ala His Ser Ser His
    515                 520                 525

caa cgc ttt ctg tat ggg ctt cct gga gag gat cag gag gag ggg agg 1689
Gln Arg Phe Leu Tyr Gly Leu Pro Gly Glu Asp Gln Glu Glu Gly Arg
530                 535                 540                 545

acc tcg gga tga actcccacca gggcctggcc acgggtggga tgagtgacca 1741
Thr Ser Gly

cagtggcctg ccagacaagg gtgtgtgtgt gtgtgtgtgt gtgtgtgtgt gtgcatgcac 1801

agcaagatgg agagggagtt cacgggtggg atgagtgggc acagtggcct gccagacaag 1861

ggtgtgtgtg tgtgtgcaca cacacaaaga tagagaggga attctggaaa ccccccacga 1921

catagagacc cgctattttc tctctcacat ctctgtattc ttgtgtatta tatggggaca 1981

cccctctcgc ggtgaga 1998

<210> 22
<211> 548
<212> PRT
<213> Capra hircus

<400> 22

Met Ser Gly Asp Thr Gly Thr Pro Asn Gln Gly Gly Thr Arg Tyr Gly
1               5                   10                  15

Ser Ile Ser Ser Pro Pro Ser Pro Gly Pro Gln Gln Ala Pro Pro Gly
            20                  25                  30

Gly Thr Tyr Leu Ser Glu Lys Ile Pro Ile Pro Asp Thr Glu Ser Gly
        35                  40                  45

Ala Phe Ser Leu Arg Lys Leu Trp Ala Phe Thr Gly Pro Gly Phe Leu
    50                  55                  60

Met Ser Ile Ala Phe Leu Asp Pro Gly Asn Ile Glu Ser Asp Leu Gln
65                  70                  75                  80

Ala Gly Ala Val Ala Gly Phe Lys Leu Leu Trp Val Leu Leu Trp Ala
                85                  90                  95

Thr Val Leu Gly Leu Leu Cys Gln Arg Leu Ala Ala Arg Leu Gly Val
            100                 105                 110

Val Thr Gly Lys Asp Leu Gly Glu Val Cys His Leu Tyr Tyr Pro Lys
        115                 120                 125

Val Pro Arg Ile Leu Leu Trp Leu Thr Ile Glu Leu Ala Ile Val Gly
    130                 135                 140

Ser Asp Met Gln Glu Val Ile Gly Thr Ala Ile Ala Phe Ser Leu Leu
145                 150                 155                 160

Ser Ala Gly Arg Ile Pro Leu Trp Gly Gly Val Leu Ile Thr Ile Val
                165                 170                 175

Asp Thr Phe Phe Phe Leu Phe Leu Asp Asn Tyr Gly Leu Arg Lys Leu
            180                 185                 190

Glu Ala Phe Phe Gly Phe Leu Ile Thr Ile Met Ala Leu Thr Phe Gly
        195                 200                 205

Tyr Glu Tyr Val Val Ala Arg Pro Ala Gln Gly Ala Leu Leu Gln Gly
    210                 215                 220

Leu Phe Leu Pro Ser Cys Pro Gly Cys Gly Gln Pro Glu Leu Leu Gln
225                 230                 235                 240

Ala Val Gly Ile Val Gly Ala Ile Ile Met Pro His Asn Ile Tyr Leu
                245                 250                 255

His Ser Ser Leu Val Lys Ser Arg Glu Val Asp Arg Ser Arg Arg Ala
            260                 265                 270

Asp Ile Arg Glu Ala Asn Met Tyr Phe Leu Ile Glu Ala Thr Ile Ala
        275                 280                 285

Leu Ser Val Ser Phe Phe Ile Asn Leu Phe Val Met Ala Val Phe Gly
    290                 295                 300

Gln Ala Phe Tyr Lys Gln Thr Asn Gln Ala Ala Phe Asn Ile Cys Ala
305                 310                 315                 320

Asn Ser Ser Leu His Asp Tyr Ala Thr Ile Phe Pro Arg Asp Asn Leu
                325                 330                 335

Thr Val Ala Val Asp Ile Tyr Gln Gly Gly Val Ile Leu Gly Cys Leu
            340                 345                 350

Phe Gly Pro Ala Ala Leu Tyr Ile Trp Ala Val Gly Leu Leu Ala Ala
        355                 360                 365

Gly Gln Ser Ser Thr Met Thr Gly Thr Tyr Ala Gly Gln Phe Val Met
    370                 375                 380

Glu Gly Phe Leu Lys Leu Arg Trp Ser Arg Phe Ala Arg Val Leu Leu
385                 390                 395                 400

Thr Arg Ser Cys Ala Ile Leu Pro Thr Val Leu Leu Ala Val Phe Arg
                405                 410                 415

Asp Leu Gln Asp Leu Ser Gly Leu Asn Asp Leu Leu Asn Val Leu Gln
            420                 425                 430

Ser Leu Leu Leu Pro Phe Ala Val Leu Pro Ile Leu Thr Phe Thr Ser
        435                 440                 445

Met Pro Ala Leu Met Gln Glu Phe Ala Asn Gly Leu Val Ser Lys Ile
    450                 455                 460

Ile Thr Ser Ser Ile Met Val Leu Val Cys Ala Val Asn Leu Tyr Phe
465                 470                 475                 480

Val Ile Ser Tyr Val Pro Ser Leu Pro His Pro Ala Tyr Phe Ser Leu
                485                 490                 495

Val Ala Leu Leu Ala Ala Ala Tyr Leu Gly Leu Thr Thr Tyr Leu Val
            500                 505                 510

Trp Thr Cys Leu Ile Thr Gln Gly Ala Thr His Leu Ala His Ser Ser
        515                 520                 525

His Gln Arg Phe Leu Tyr Gly Leu Pro Gly Glu Asp Gln Glu Glu Gly
    530                 535                 540

Arg Thr Ser Gly
545

<210> 23
<211> 548
<212> PRT
<213> Ovis aries

<400> 23

Met Ser Gly Asp Thr Gly Thr Pro Asn Gln Gly Gly Thr Arg Tyr Gly
1               5                   10                  15

Ser Ile Ser Ser Pro Pro Ser Pro Gly Pro Gln Gln Ala Pro Pro Gly
            20                  25                  30

Gly Thr Tyr Leu Ser Glu Lys Ile Pro Ile Pro Asp Thr Glu Ser Gly
        35                  40                  45

Ala Phe Ser Leu Arg Lys Leu Trp Ala Phe Thr Gly Pro Gly Phe Leu
    50                  55                  60

Met Ser Ile Ala Phe Leu Asp Pro Gly Asn Ile Glu Ser Asp Leu Gln
65                  70                  75                  80

Ala Gly Ala Val Ala Gly Phe Lys Leu Leu Trp Val Leu Leu Trp Ala
                85                  90                  95

Thr Val Leu Gly Leu Leu Cys Gln Arg Leu Ala Ala Arg Leu Gly Val
            100                 105                 110

Val Thr Gly Lys Asp Leu Gly Glu Val Cys His Leu Tyr Tyr Pro Lys
        115                 120                 125

Val Pro Arg Thr Leu Leu Trp Leu Thr Ile Glu Leu Ala Ile Val Gly
    130                 135                 140

Ser Asp Met Gln Glu Val Ile Gly Thr Ala Ile Ala Phe Ser Leu Leu
145                 150                 155                 160

Ser Ala Gly Arg Ile Pro Leu Trp Gly Gly Val Leu Ile Thr Ile Val
                165                 170                 175

Asp Thr Phe Phe Phe Leu Phe Leu Asp Asn Tyr Gly Leu Arg Lys Leu
            180                 185                 190

Glu Ala Phe Phe Gly Phe Leu Ile Thr Ile Met Ala Leu Thr Phe Gly
        195                 200                 205

Tyr Glu Tyr Val Val Ala Arg Pro Ala Gln Gly Ala Leu Leu Gln Gly
    210                 215                 220

Leu Phe Leu Pro Ser Cys Pro Gly Cys Gly Gln Pro Glu Leu Leu Gln
225                 230                 235                 240

Ala Val Gly Ile Val Gly Ala Ile Ile Met Pro His Asn Ile Tyr Leu
                245                 250                 255

His Ser Ser Leu Val Lys Ser Arg Glu Val Asp Arg Ser Arg Arg Ala
            260                 265                 270

Asp Ile Arg Glu Ala Asn Met Tyr Phe Leu Ile Glu Ala Thr Ile Ala
        275                 280                 285

Leu Ser Val Ser Phe Phe Ile Asn Leu Phe Val Met Ala Val Phe Gly
    290                 295                 300

Gln Ala Phe Tyr Lys Gln Thr Asn Gln Ala Ala Phe Asn Ile Cys Ala
305                 310                 315                 320

Asn Ser Ser Leu His Asp Tyr Ala Thr Ile Phe Pro Arg Asp Asn Leu
                325                 330                 335

Thr Val Ala Val Asp Ile Tyr Gln Gly Gly Val Ile Leu Gly Cys Leu
            340                 345                 350

Phe Gly Pro Ala Ala Leu Tyr Ile Trp Ala Val Gly Leu Leu Ala Ala
        355                 360                 365

Gly Gln Ser Ser Thr Met Thr Gly Thr Tyr Ala Gly Gln Phe Val Met
    370                 375                 380

Glu Gly Phe Leu Lys Leu Arg Trp Ser Arg Phe Ala Arg Val Leu Leu
385                 390                 395                 400

Thr Arg Ser Cys Ala Ile Leu Pro Thr Val Leu Leu Ala Val Phe Arg
                405                 410                 415

Asp Leu Gln Asp Leu Ser Gly Leu Asn His Val Leu Asn Val Leu Gln
            420                 425                 430

Ser Leu Leu Leu Pro Phe Ala Val Leu Pro Ile Leu Thr Phe Thr Ser
        435                 440                 445

Met Pro Ala Leu Met Gln Glu Phe Ala Asn Gly Leu Val Ser Lys Ile
    450                 455                 460

Ile Thr Ser Ser Ile Met Val Leu Val Cys Ala Val Asn Leu Tyr Phe
465                 470                 475                 480

Val Ile Ser Tyr Val Pro Ser Leu Pro His Pro Ala Tyr Phe Ser Leu
                485                 490                 495

Val Ala Leu Leu Ala Ala Ala Tyr Leu Gly Leu Thr Thr Tyr Leu Val
            500                 505                 510

Trp Thr Cys Leu Ile Thr Gln Gly Ala Thr Arg Leu Ala His Ser Ser
        515                 520                 525

His Gln Arg Phe Leu Tyr Gly Leu Pro Gly Glu Asp Gln Glu Glu Gly
    530                 535                 540

Arg Thr Ser Gly
545

<210>   24
<211>   548
<212>   PRT

<213>  Bos taurus

<400>  24

Met Ser Gly Asp Thr Gly Pro Pro Lys Gln Gly Gly Thr Arg Tyr Gly
1               5                   10                  15

Ser Ile Ser Ser Pro Pro Ser Pro Glu Pro Gln Gln Ala Pro Pro Gly
            20                  25                  30

Gly Thr Tyr Leu Ser Glu Lys Ile Pro Ile Pro Asp Thr Glu Ser Gly
        35                  40                  45

Thr Phe Ser Leu Arg Lys Leu Trp Ala Phe Thr Gly Pro Gly Phe Leu
    50                  55                  60

Met Ser Ile Ala Phe Leu Asp Pro Gly Asn Ile Glu Ser Asp Leu Gln
65                  70                  75                  80

Ala Gly Ala Val Ala Gly Phe Lys Leu Leu Trp Val Leu Leu Trp Ala
                85                  90                  95

Thr Val Leu Gly Leu Leu Cys Gln Arg Leu Ala Ala Arg Leu Gly Val
            100                 105                 110

Val Thr Gly Lys Asp Leu Gly Glu Val Cys His Leu Tyr Tyr Pro Lys
        115                 120                 125

Val Pro Arg Ile Leu Leu Trp Leu Thr Ile Glu Leu Ala Ile Val Gly
    130                 135                 140

Ser Asp Met Gln Glu Val Ile Gly Thr Ala Ile Ala Phe Ser Leu Leu
145                 150                 155                 160

Ser Ala Gly Arg Ile Pro Leu Trp Gly Gly Val Leu Ile Thr Val Val
                165                 170                 175

Asp Thr Phe Phe Phe Leu Phe Leu Asp Asn Tyr Gly Leu Arg Lys Leu
            180                 185                 190

Glu Ala Phe Phe Gly Phe Leu Ile Thr Ile Met Ala Leu Thr Phe Gly
        195                 200                 205

Tyr Glu Tyr Val Val Ala Gln Pro Ala Gln Gly Ala Leu Leu Gln Gly
    210                 215                 220

Leu Phe Leu Pro Ser Cys Pro Gly Cys Gly Gln Pro Glu Leu Leu Gln
225                 230                 235                 240

Ala Val Gly Ile Ile Gly Ala Ile Ile Met Pro His Asn Ile Tyr Leu
                245                 250                 255

His Ser Ser Leu Val Lys Ser Arg Glu Val Asp Arg Ser Arg Arg Ala
            260                 265                 270

Asp Ile Arg Glu Ala Asn Met Tyr Phe Leu Ile Glu Ala Thr Ile Ala
        275                 280                 285

Leu Ser Val Ser Phe Leu Ile Asn Leu Phe Val Met Ala Val Phe Gly
    290                 295                 300

Gln Ala Phe Tyr Lys Gln Thr Asn Gln Ala Ala Phe Asn Ile Cys Ala
305                 310                 315                 320

Asp Ser Ser Leu His Asp Tyr Ala Pro Ile Phe Pro Arg Asn Asn Leu
                325                 330                 335

Thr Val Ala Val Asp Ile Tyr Gln Gly Gly Val Ile Leu Gly Cys Leu
            340                 345                 350

Phe Gly Pro Pro Ala Leu Tyr Ile Trp Ala Val Gly Leu Leu Ala Ala
        355                 360                 365

Gly Gln Ser Ser Thr Met Thr Gly Thr Tyr Ala Gly Gln Phe Val Met
    370                 375                 380

Glu Gly Phe Leu Lys Leu Arg Trp Ser Arg Phe Ala Arg Val Leu Leu
385                 390                 395                 400

Thr Arg Ser Cys Ala Ile Leu Pro Thr Val Leu Leu Ala Val Phe Arg
                405                 410                 415

Asp Leu Arg Asp Leu Ser Gly Leu Asn Asp Leu Leu Asn Val Leu Gln
            420                 425                 430

Ser Leu Leu Leu Pro Phe Ala Val Leu Pro Ile Leu Thr Phe Thr Ser
        435                 440                 445

Met Pro Ala Leu Met Gln Glu Phe Ala Asn Gly Leu Val Ser Lys Val
    450                 455                 460

Ile Thr Ser Ser Ile Met Val Leu Val Cys Ala Val Asn Leu Tyr Phe
465                 470                 475                 480

Val Ile Ser Tyr Leu Pro Ser Leu Pro His Pro Ala Tyr Phe Ser Leu
                485                 490                 495

Val Ala Leu Leu Ala Ala Ala Tyr Leu Gly Leu Thr Thr Tyr Leu Val
            500                 505                 510

Trp Thr Cys Leu Ile Thr Gln Gly Ala Thr Leu Leu Ala His Ser Ser
        515                 520                 525

His Gln Arg Phe Leu Tyr Gly Leu Pro Glu Glu Asp Gln Glu Lys Gly
    530                 535                 540

Arg Thr Ser Gly
545